# EUROPEAN PATENT APPLICATION

(11) **EP 4 310 100 A1**
(43) Date of publication of application: **24.01.2024**
(21) Application number: 21931548.8
(22) Date of filing: 18.03.2021
(51) Int. Cl.: C07K 16/18, C12N 15/13, G01N 33/574

(54) **MONOCLONAL ANTIBODY SPECIFICALLY BINDING TO SUGAR CHAIN IN WHICH A TERMINAL SIALIC ACID RESIDUE IS BOUND TO GALACTOSE WITH ?2,6 LINKAGE, AND METHOD FOR MEASURING SUGAR CHAIN IN WHICH TERMINAL SIALIC ACID RESIDUE IS BOUND TO GALACTOSE WITH ?2,6 LINKAGE**

(71) Applicant: HIROSAKI UNIVERSITY, Hirosaki-shi, Aomori 036-8560 (JP); System Instruments Co., Ltd., Hachioji-shi, Tokyo 192-0031 (JP)
(72) Inventor: OHYAMA Chikara, Hirosaki-shi, Aomori 036-8560 (JP); YONEYAMA Tohru, Hirosaki-shi, Aomori 036-8560 (JP); HAMADA Kazuyuki, Hachioji-shi, Tokyo 192-0031 (JP)
(74) Representative: Regimbeau
(86) International application number: PCT/JP2021/011037
(87) International publication number: WO 2022/195797

(57) **Abstract**

A monoclonal antibody or an antibody fragment thereof is provided which specifically binds to a sugar chain in which a terminal sialic acid residue is bound to galactose with α2,6 linkage, and which does not bind to a sugar chain to which galactose having no sialic acid bound to a terminal is bound.

## Description

### TECHNICAL FIELD

The present invention relates to a monoclonal antibody specifically binding to a sugar chain in which a terminal sialic acid residue is bound to galactose with α2,6 linkage, and a method for measuring a sugar chain in which a terminal sialic acid residue is bound to galactose with α2,6 linkage.

### BACKGROUND ART

Regarding a sugar chain of prostate specific antigen (PSA), which is known as a marker for prostate cancer, it is known that a double-stranded N-type sugar chain in which sialic acid is bound to galactose with α2,6 linkage at the terminal and an N-type sugar chain in which sialic acid at the terminal is bound to galactose with α2,3 linkage are present, and that the number of sugar chains that are bound to galactose with α2,3 linkage increases as compared with a case of α2,6 linkage, in association with canceration (Non-Patent Document 1). Therefore, the detection of the sugar chain in which the terminal sialic acid residue is bound to galactose with α2,3 linkage and the concurrent detection of the sugar chain in which the terminal sialic acid residue is bound to galactose with α2,6 linkage are useful for detecting canceration of PSA.

Sambucus nigra lectin is known as a probe capable of detecting a sugar chain in which a terminal sialic acid residue is bound to galactose with α2,6 linkage, and it is used as a probe for detecting a sugar chain containing sialic acid (Patent Document 1). In addition, a mouse monoclonal antibody that recognizes a sugar chain in which a terminal sialic acid residue is bound to galactose with α2,6 linkage has been reported where a glycolipid having a sugar chain in which a terminal sialic acid residue is bound to galactose with α2,6 linkage is used as an immunogen (Patent Document 2).

### Citation List

### Patent Documents

[Patent Document 1]
   Published Japanese Translation No. 2011-529184 of the PCT International Publication
[Patent Document 2]
   Japanese Patent No. 6172687

### [Non-Patent Document]

[Non-Patent Document 1]
Tajiri M, Ohyama C, Wada Y, Glycobiology, 2008; 18: 2-8

### DISCLOSURE OF INVENTION

### Technical Problem

However, since Sambucus nigra lectin is a natural product derived from a plant as a raw material and the bindability thereof changes depending on the product lot, the clinical application of a tumor biomarker using this as a probe is difficult. Although the development of recombinant lectins is underway, they have not yet been put into practical use.

In addition, an antibody that recognizes, with higher sensitivity, a sugar chain in which a terminal sialic acid residue is bound to galactose with α2,6 linkage is required for the detection of canceration of PSA.

The present invention has been made in consideration of the above problems, and an object thereof is to provide a monoclonal antibody having high bindability to a sugar chain in which a terminal sialic acid residue is bound to galactose with α2,6 linkage, and a method for measuring a sugar chain in which a terminal sialic acid residue is bound to galactose with α2,6 linkage.

### Solution to Problem

The inventors of the present invention found that a monoclonal antibody obtained by immunizing a rabbit with Siaα2-6Galβ1-4GlcNAc-BSA as an immunogen, isolating an obtained positive rabbit B cell, acquiring an antibody gene by single-cell PCR, and introducing the antibody gene into a host cell binds, with high bindability, to a sugar chain in which a terminal sialic acid residue is bound to galactose with α2,6 linkage, thereby completing the present invention.

The present invention includes the following aspects.

[1] A monoclonal antibody or an antibody fragment thereof which specifically binds to a sugar chain in which a terminal sialic acid residue is bound to galactose with α2,6 linkage, and which does not bind to a sugar chain to which galactose having no sialic acid bound to a terminal is bound.
[2] The monoclonal antibody or the antibody fragment thereof according to [1] in which a dissociation constant with respect to the sugar chain in which the terminal sialic acid residue is bound to galactose with α2,6 linkage is 1.0 × 10⁻⁸ or less.
[3] The monoclonal antibody or the antibody fragment thereof according to [1] or [2] in which the bindability to the sugar chain in which the terminal sialic acid residue is bound to galactose with α2,6 linkage is 20 times or more with respect to the bindability to a sugar chain in which a terminal sialic acid residue is bound to galactose with α2,3 linkage.
[4] The monoclonal antibody or the antibody fragment thereof according to any one of [1] to [3] in which an amino acid sequence of a complementarity determining region (hereinafter, also referred to as CDR) 1 of a heavy chain variable region (hereinafter, also referred to as VH) of the antibody or the antibody fragment thereof includes an amino acid sequence set forth in SEQ ID NO: 1, an amino acid sequence of CDR2 of the VH includes an amino acid sequence set forth in SEQ ID NO: 2, an amino acid sequence of CDR3 of VH includes an amino acid sequence set forth in SEQ ID NO: 3, an amino acid sequence of CDR1 of a light chain variable region (hereinafter, also referred to as VL) includes an amino acid sequence set forth in SEQ ID NO: 4, an amino acid sequence of CDR2 of VL includes an amino acid sequence set forth in SEQ ID NO: 5, and an amino acid sequence of CDR3 of VL includes an amino acid sequence set forth in SEQ ID NO: 6.
[5] The monoclonal antibody or the antibody fragment thereof according to [4] in which an amino acid sequence of the VH of the antibody or the antibody fragment thereof includes an amino acid sequence set forth in SEQ ID NO: 13, and an amino acid sequence of VL includes an amino acid sequence set forth in SEQ ID NO: 14.
[6] The monoclonal antibody or the antibody fragment thereof according to any one of [1] to [3] in which an amino acid sequence of CDR1 of the VH of the antibody or the antibody fragment thereof includes an amino acid sequence set forth in SEQ ID NO: 7, an amino acid sequence of CDR2 of the VH includes an amino acid sequence set forth in SEQ ID NO: 8, the amino acid sequence of CDR3 of VH includes an amino acid sequence set forth in SEQ ID NO: 9, an amino acid sequence of CDR1 of VL includes an amino acid sequence set forth in SEQ ID NO: 10, an amino acid sequence of CDR2 of VL includes an amino acid sequence set forth in SEQ ID NO: 11, and the amino acid sequence of CDR3 of VL includes an amino acid sequence set forth in SEQ ID NO: 12.
[7] The monoclonal antibody or the antibody fragment thereof according to [6] in which an amino acid sequence of the VH of the antibody or the antibody fragment thereof includes an amino acid sequence set forth in SEQ ID NO: 15, and an amino acid sequence of VL includes an amino acid sequence set forth in SEQ ID NO: 16.
[8] A method for measuring a sugar chain in which a terminal sialic acid residue is bound to galactose with α2,6 linkage, the method including using the monoclonal antibody or the antibody fragment thereof according to any one of [1] to [7].

### Advantageous Effects of Invention

According to the present invention, it is possible to provide a monoclonal antibody having high bindability to a sugar chain (hereinafter, also referred to as an α2,6 sugar chain) in which a terminal sialic acid residue is bound to galactose with α2,6 linkage, and a method for measuring an α2,6 sugar chain.

### BRIEF DESCRIPTION OF DRAWINGS

FIG. 1 is a graph showing results of confirming bindability of an antibody obtained in Example 1 with respect to a sugar chain in which a terminal sialic acid residue is bound to galactose with α2,3 linkage (hereinafter, also referred to as an α2,3 sugar chain) and an α2,6 sugar chain by ELISA.
FIG. 2A is a graph showing results of a sensorgram of an 8A2 antibody to a biosensor on which an α2,6 sugar chain is immobilized, in Example 2.
FIG. 2B is a graph showing results of a sensorgram of the 8A2 antibody to a biosensor on which an α2,3 sugar chain is immobilized, in Example 2.
FIG. 2C is a graph showing results of a sensorgram of the 8A2 antibody to a biosensor on which a sugar chain to which galactose having no sialic acid bound to a terminal is bound (hereinafter, also referred to as a galactose-bound sugar chain) is immobilized, in Example 2.
FIG. 3A is a graph showing results of a sensorgram of an 8B9 antibody to a biosensor on which an α2,6 sugar chain is immobilized, in Example 2.
FIG. 3B is a graph showing results of a sensorgram of an 8B9 antibody to a biosensor on which an α2,3 sugar chain is immobilized, in Example 2.
FIG. 3C is a graph showing the results of a sensorgram of an 8B9 antibody to a biosensor on which a galactose-bound sugar chain is immobilized, in Example 2.

### BEST MODE FOR CARRYING OUT THE INVENTION

Hereinafter, embodiments according to the present invention will be described in detail.

### <Monoclonal antibody and antibody fragment thereof>

In the present specification, "antibody" refers to a full-length immunoglobulin molecule that exists in nature or is produced by genetic recombination technology, and "antibody fragment" refers to an antigen-binding fragment of such an immunoglobulin molecule. Such an antibody and antibody fragment can be prepared using a conventional technology. Examples of the antibody fragment include F(ab')₂, F(ab)₂, Fab', Fab, Fv, scFv, variants thereof, a fusion protein or peptide including an antibody portion, and a modified structure other than an immunoglobulin molecule including an α2,3 sugar chain-binding site.

In the present specification, the description that an antibody "specifically binds" means that the antibody substantially does not bind to a sugar chain that is different from the sugar chain in which the terminal sialic acid residue is bound to galactose with α2,6 linkage but binds to an α2,6 sugar chain. Further, in the present specification, the "α2,6 sugar chain" is also referred to as "Siaα2-6Glβ1-4GlcNAc".

In the present invention, "monoclonal antibody" means an antibody obtained from a substantially homogeneous population, and an individual antibody contained in the population is identical except for a possible natural mutant that may be present. The monoclonal antibody is an antibody exhibiting one binding specificity and affinity for a specific epitope of an antigen. The modifier "monoclonal" indicates the properties of the antibody obtained from a substantially homogeneous antibody population, and it is not to be construed as being limited by requiring production of the antibody to a specific method.

The "heavy chain" of an antibody used in the present specification refers to a larger one of the two types of polypeptide chains present in all antibody molecules in the conformation present in nature. The "light chain" of an antibody used in the present specification refers to a smaller one of the two types of polypeptide chains present in all antibody molecules in the conformation present in nature.

Here, the complementarity determining region (CDR) is composed of a heavy chain complementarity determining region and a light chain complementarity determining region. Each of the variable regions of the heavy chain and the light chain consists of three CDRs and four framework regions (FRs) connected by the CDRs. The CDRs in each chain are held in the vicinity by the FRs, and contribute to the formation of an antigen-binding site of the antibody, together with the CDRs in other chains.

Technologies for determining CDRs include, but are not limited to, (1) an approach based on heterologous sequence variability (for example, Kabat et al. Sequences of Proteins of Immunological interest, 5th ed., 1991, National Institutes of Health, Bethesda MD); and (2) an approach based on crystallographic studies of the antigen-antibody complex (Al-lazikani et al., J. Molec. Biol. 273, 927-948, 1997), for example. These approaches and other approaches may be used in combination.

The monoclonal antibody or the antibody fragment thereof according to the present invention is a monoclonal antibody or an antibody fragment thereof which specifically binds to an α2,6 sugar chain and does not bind to a sugar chain to which galactose having no sialic acid bound to a terminal is bound. Hereinafter, the monoclonal antibody according to the present invention is also referred to as an anti-α2,6 sugar chain monoclonal antibody.

The anti-α2,6 sugar chain monoclonal antibody according to the present invention may be a human antibody or may be a non-human animal antibody. Examples of the non-human animal include a mouse, a rat, a hamster, a rabbit, a goat, a sheep, and a chicken, where a rabbit monoclonal antibody is preferable since it has high bindability to an antigen.

The anti-α2,6 sugar chain monoclonal antibody or an antibody fragment thereof according to the present invention is a monoclonal antibody or an antibody fragment thereof which does not bind to a galactose-bound sugar chain.

The bindability of the anti-α2,6 sugar chain monoclonal antibody or an antibody fragment thereof according to the present invention to the α2,6 sugar chain is preferably higher than the bindability to the α2,3 sugar chain. For example, the bindability to the α2,6 sugar chain is 20 times or more, preferably 30 times or more, more preferably 40 times or more, still more preferably 50 times or more, and particularly preferably 80 times or more, with respect to the bindability to the α2,3 sugar chain.

The bindability of the anti-α2,6 sugar chain monoclonal antibody or an antibody fragment thereof according to the present invention to an antigen such as an α2,6 sugar chain can be indicated by a dissociation constant (a KD value). The unit of KD is M, and the higher the bindability, the lower the KD value.

The KD value of the anti-α2,6 sugar chain monoclonal antibody or an antibody fragment thereof according to the present invention with respect to the α2,6 sugar chain is preferably 1.0 × 10⁻⁸ or less, more preferably 5.0 × 10⁻⁹ or less, still more preferably 1.0 × 10⁻⁹ or less, and particularly preferably 5.0 × 10⁻¹⁰ or less. For example, it may be 8.5 × 10⁻⁹ or less, 8.0 × 10⁻⁹ or less, 7.0 × 10⁻⁹ or less, 6.0 × 10⁻⁹ or less, 4.0 × 10⁻⁹ or less, 3.0 × 10⁻⁹ or less, 2.0 × 10⁻⁹ or less, 9.0 × 10⁻¹⁰ or less, 8.0 × 10⁻¹⁰ or less, 7.0 × 10⁻¹⁰ or less, 6.0 × 10⁻¹⁰ or less, 4.0 × 10⁻¹⁰ or less, or 3.0 × 10⁻¹⁰ or less.

The KD value can be calculated using, for example, a biosensor on which a sugar chain serving as an antigen is immobilized. Specifically, a sugar chain serving as an antigen is immobilized on a biosensor and immersed in an antibody solution to allow the antibody to bind to the antigen immobilized on the biosensor. Then, the biosensor is immersed in a buffer solution such as phosphate-buffered saline (PBS), a change in the wavelength shift Δλ caused by the change in the number of antibodies bound to the biosensor or the number of antibodies dissociated from the biosensor is measured, and then the KD value can be calculated from the sensorgram obtained when the concentration of the antibody is changed.

The anti-α2,6 sugar chain monoclonal antibody or an antibody fragment thereof according to the present invention can be produced by using a known method. Specifically, first, a conjugate of an α2,6 sugar chain and a carrier protein is used as an immunogen to immunize a non-human animal, the bindability to an antigen is checked by ELISA for lymphocytes of the immunized non-human animal, and then a lymphocyte having high bindability to the α2,6 sugar chain is selected. Examples of the non-human animal to be immunized include a mouse, a rat, a hamster, a rabbit, a goat, sheep, and a chicken. Next, the antibody gene is obtained from the selected lymphocyte by the single-cell PCR method and amplified by the PCR method. For the PCR amplification product, the bindability to the antigen is confirmed by ELISA. A PCR amplification product having high bindability to the α2,6 sugar chain is transfected into cells such as human embryonic kidney cells 293, the culture supernatant containing the secreted antibody is recovered, and the bindability of the recovered sample to the antigen is confirmed by ELISA, thereby obtaining a clone having high bindability to the α2,6 sugar chain. The antibody gene is obtained from the obtained clone and inserted into a vector to obtain an antibody-producing cell. The obtained antibody-producing cell is cultured to generate and accumulate the anti-α2,6 sugar chain monoclonal antibody or an antibody fragment thereof according to the present invention, and the anti-α2,6 sugar chain monoclonal antibody or an antibody fragment thereof according to the present invention can be produced from the culture.

Examples of the anti-α2,6 sugar chain monoclonal antibody or an antibody fragment thereof according to the present invention include an anti-α2,6 sugar chain monoclonal antibody 8A2 (hereinafter, also referred to as an 8A2 antibody) or an antibody fragment thereof which includes an amino acid sequence of CDR1 of VH includes an amino acid sequence set forth in SEQ ID NO: 1, an amino acid sequence of CDR2 of the VH includes an amino acid sequence set forth in SEQ ID NO: 2, an amino acid sequence of CDR3 of VH includes an amino acid sequence set forth in SEQ ID NO: 3, an amino acid sequence of CDR1 of VL includes an amino acid sequence set forth in SEQ ID NO: 4, an amino acid sequence of CDR2 of VL includes an amino acid sequence set forth in SEQ ID NO: 5, and an amino acid sequence of CDR3 of VL includes an amino acid sequence set forth in SEQ ID NO: 6.

The antibody 8A2 includes VH including an amino acid sequence represented by SEQ ID NO: 13 and VL including an amino acid sequence represented by SEQ ID NO: 14.

In addition, examples of the anti-α2,6 sugar chain monoclonal antibody or an antibody fragment thereof according to the present invention include an anti-α2,6 sugar chain monoclonal antibody 8B9 (hereinafter, also referred to as an 8B9 antibody) or an antibody fragment thereof which includes an amino acid sequence of CDR1 of VH includes an amino acid sequence set forth in SEQ ID NO: 7, an amino acid sequence of CDR2 of the VH includes an amino acid sequence set forth in SEQ ID NO: 8, an amino acid sequence of CDR3 of VH includes an amino acid sequence set forth in SEQ ID NO: 9, an amino acid sequence of CDR1 of VL includes an amino acid sequence set forth in SEQ ID NO: 10, an amino acid sequence of CDR2 of VL includes an amino acid sequence set forth in SEQ ID NO: 11, and an amino acid sequence of CDR3 of VL includes an amino acid sequence set forth in SEQ ID NO: 12.

The antibody 8B9 includes VH including an amino acid sequence represented by SEQ ID NO: 15 and VL including an amino acid sequence represented by SEQ ID NO: 16.

The amino acid sequences set forth in SEQ ID NO: 1 to SEQ ID NO: 12 are shown in Table 1.

**[Table 1]**

| Antibody No. | SEQ ID NO | Variable region | CDR | Amino acid sequence |
|---|---|---|---|---|
| 8A2 | 1 | VH | 1 | ASGFSFSN |
| | 2 | VH | 2 | CIVTRDSN |
| | 3 | VH | 3 | CARWNGSFRL |
| | 4 | VL | 1 | QSVYNKNW |
| | 5 | VL | 2 | AAS |
| | 6 | VL | 3 | QGDFSGNIHS |
| 8B9 | 7 | VH | 1 | ASGFSFSG |
| | 8 | VH | 2 | CFWFSSG |
| | 9 | VH | 3 | CARRGDNTYGL |
| | 10 | VL | 1 | QSISSY |
| | 11 | VL | 2 | YAS |
| | 12 | VL | 3 | QNTARSSNNGDP |

The amino acid sequences set forth in SEQ ID NO: 13 to SEQ ID NO: 16 are shown in Table 2.

**[Table 2]**

| Antibody No. | SEQ ID NO | Variable region | Amino acid sequence |
|---|---|---|---|
| 8A2 | 13 | VH | QSLEESGGDLVKPGASLTLTCTASGFSFSNNYWICWVRQAPGKGLE WIGCIVTRDSNTAYANWAKGRLTISKTSSTTVTLQMTSLTVADTATY FCARVVNGSFRLWGPGTLVTVSS |
| | 14 | VL | AQVLTQTASPVSAPVGGTVTINCQSSQSVYNKNWLSWYQQKPGQP PKLLINAASNLASGVPSRFSGSGSGTQFTLTISDLECDDAATYYCQG DFSGNIHSFGGGTEVVVK |
| 8B9 | 15 | VH | QEQLVESGGGLVQPEGSLTLTCTASGFSFSGIYAMCWVRQAPGKGL EWIACFWFSSGSTYYATWAKGRFTISKTSSTTVTLQMTSLTAADTAT YFCARRGDNTYGLWGPGTLVTVSS |
| | 16 | VL | ALVMTQTPASVSAAVGGTVTIKCQASQSISSYCSWYQQKPGQRPKL LIYYASKLASGVPSRFKGSGSGTEFTLTISDLECADAATYYCQNTAR SSNNGDPFGGGTEVVVK |

The anti-α2,6 sugar chain monoclonal antibody according to the present invention or an antibody fragment thereof which includes CDR1 to CDR3 of VH and CDR1 to CDR3 of VL can be produced by using a known genetic recombination technology. Specifically, it is possible to produce this antibody or the antibody fragment thereof by incorporating each of the genes encoding CDR1 to CDR3 of VH and CDR1 to CDR3 of VL into a vector including the genes encoding each of the FR of the antibody and the constant region of the antibody, introducing this into a host cell, and transforming the host cell to obtain a cell expressing the antibody, and culturing the cell. The cell used in the preparation, the kind of vector, the kind of cell, the culture conditions, and the like are within the technical range of those skilled in the art, and appropriate conditions can be appropriately set.

The monoclonal antibody or the antibody fragment thereof according to the present invention also includes a monoclonal antibody or an antibody fragment thereof in which in the amino acid sequences of CDR1 to 3 of VH and VL, one or more amino acids are deleted, added, substituted, or inserted and which has the same specificity as the monoclonal antibody or the antibody fragment thereof according to the present invention.

The number of amino acids to be deleted, substituted, inserted, and/or added is one or more, although the number thereof is not particularly limited; however, it is such a number that deletion, substitution, or addition can be carried out by a well-known technique such as a site-specific mutagenesis method [Molecular Cloning 2nd Edition, Cold Spring Harbor Laboratory Press (1989), Current Protocols in Molecular Biology, John Wiley & Sons (1987-1997), Nucleic Acids Research 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985)]. For example, it is preferably 1 to several tens of amino acids, more preferably 1 to 20 amino acids, still more preferably 1 to 10 amino acids, and particularly preferably 1 to 5 amino acids.

### <Measuring method>

The method for measuring an α2,6 sugar chain according to the present invention is a method for measuring an α2,6 sugar chain using the anti-α2,6 sugar chain monoclonal antibody or an antibody fragment thereof according to the present invention. Examples of the method for measuring an α2,6 sugar chain using the anti-α2,6 sugar chain monoclonal antibody or an antibody fragment thereof according to the present invention include an immunological measuring method for an α2,6 sugar chain in a specimen in which an α2,6 sugar chain in a specimen is reacted with the anti-α2,6 sugar chain monoclonal antibody or an antibody fragment thereof according to the present invention, a labeled antibody or a labeled antibody fragment having a label bound to the anti-α2,6 sugar chain monoclonal antibody or an antibody fragment thereof is subsequently added to generate an immune complex consisting of the α2,6 sugar chain, the anti-α2,6 sugar chain monoclonal antibody or an antibody fragment thereof, and the labeled antibody or the labeled antibody fragment, and the amount of the label in the generated immune complex is measured.

In the measuring method using the anti-α2,6 sugar chain monoclonal antibody or an antibody fragment thereof according to the present invention, examples of the specimen include blood such as serum, plasma or whole blood, lymphatic fluid, tissue fluid, cerebrospinal fluid, body cavity fluid, digestive juice, nasal secretions, tears, sweat, and urine of animals including humans. Furthermore, the specimen may be the specimen itself collected from a subject or a product obtained by subjecting the collected specimen to usual treatments such as dilution and concentration. In addition, the specimen may be a specimen collected or prepared at the time of carrying out the measuring method or may be a specimen collected or prepared in advance and stored.

The immunological measuring method can be classified into an enzyme immunoassay (EIA or ELISA), a radioimmunoassay (RIA), a fluorescence immunoassay (FIA), a fluorescence polarization immunoassay (FPIA), a chemiluminescence immunoassay (CLIA), an electrochemiluminescence immunoassay or the like depending on the label of the labeled detection antibody, and any one of these can be used as the measuring method according to the present invention. However, ELISA is preferable since it is possible to conveniently and quickly measure the detection target.

The α2,6 sugar chain in the specimen can be measured by washing the immune complex and then measuring the label in the immune complex. For example, in a case of ELISA, the α2,6 sugar chain in the specimen can be measured by reacting an enzyme, which is a label, with a substrate of the enzyme and measuring the absorbance of a colored product (a sandwich method). Furthermore, the α2,6 sugar chain in the specimen can also be measured by reacting the anti-α2,6 sugar chain monoclonal antibody or an antibody fragment thereof according to the present invention immobilized on a solid support with the α2,6 sugar chain in the specimen, subsequently adding an unlabeled anti-α2,6 sugar chain monoclonal antibody or an antibody fragment thereof (a primary antibody), further adding a labeled secondary antibody obtained by enzymatic labeling of an antibody (a secondary antibody) against this unlabeled antibody or the antibody fragment thereof with enzyme, and measuring the label of the secondary antibody. In addition, the α2,6 sugar chain in the specimen can be measured by labeling the secondary antibody with biotin, allowing avidin or streptavidin labeled with an enzyme or the like to bind to biotin, labeling the secondary antibody with an enzyme and the like, and measuring the label of the secondary antibody.

The α2,6 sugar chain in the specimen can also be measured by adding an unlabeled anti-α2,6 sugar chain monoclonal antibody or an antibody fragment thereof (a primary antibody) to an α2,6 sugar chain or a conjugate of an α2,6 sugar chain and a protein such as BSA immobilized on a solid support to generate an immune complex consisting of the α2,6 sugar chain and the primary antibody on the solid support, adding the specimen, further adding a secondary antibody obtained by labeling an antibody (a secondary antibody) against this unlabeled antibody, and measuring the label of the labeled secondary antibody (a competitive method).

The solid support is not particularly limited as long as it can reliably hold an antibody or an antibody fragment. Examples of the preferred material of the solid support include polymer materials such as polystyrene, polycarbonate, polyvinyl toluene, polypropylene, polyethylene, polyvinyl chloride, nylon, polymethacrylate, gelatin, agarose, cellulose, nitrocellulose, cellulose acetate, acetyl cellulose, and polyethylene terephthalate, glass, ceramics, magnetic particles, metal. Examples of the preferred shape of the solid support include fine particles such as a tube, a bead, a plate, and latex, sticks.

As the label, it is possible to use an enzyme such as peroxidase and alkaline phosphatase in ELISA, a radioactive substance such as ¹²⁵I, ¹³¹I, ³⁵S, and ³H in the RIA method, and a fluorescent substance such as fluorescein isothiocyanate, rhodamine, dansyl chloride, phycoerythrin, tetramethyl rhodamine isothiocyanate, and a nearinfrared fluorescent material in the FPIA method, an enzyme such as luciferase and β-galactosidase and a luminescent substrate that is converted into a luminescent substance by each enzyme, and a luminescent substance such as luciferin and aequorin in the CLIA method. In addition, nanoparticles such as colloidal gold and quantum dots can be used as labels.

In ELISA, as the substrate of the enzyme which serves as a label, in a case where the enzyme is peroxidase, 3,3'-diaminobenzidine (DAB), 3,3',5,5'-tetramethylbenzidine (TMB), O-phenylenediamine (OPD), and the like can be used, and in a case where the enzyme is alkaline phosphatase, p-nitrophenyl phosphate (pNPP) and the like can be used.

### EXAMPLES

Hereinafter, the present invention will be described in more detail with reference to Examples; however, the present invention is not limited to these Examples.

### [Example 1]

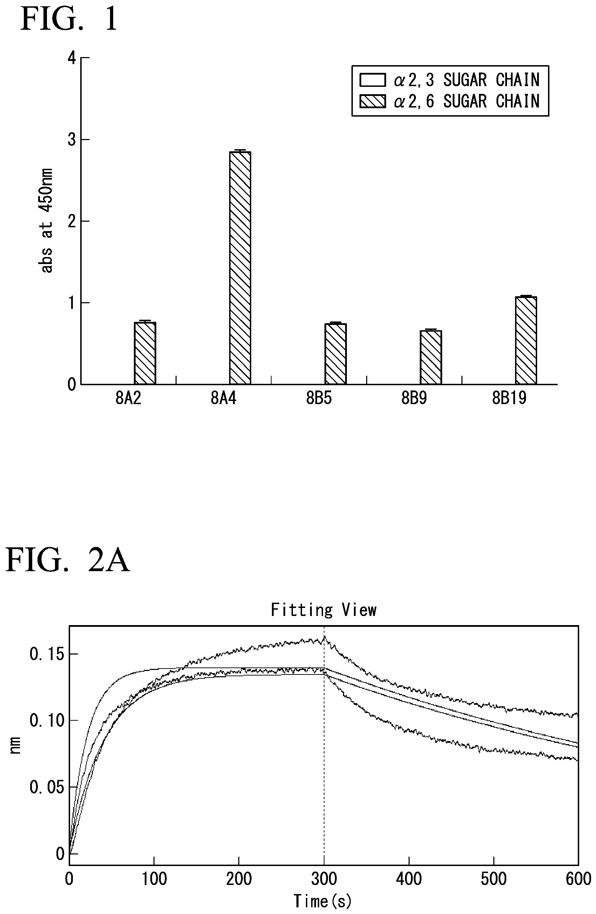

A rabbit (slc: JW/CSK, 13 weeks old) was immunized using Siaα2-6Galβ1-4GlcNAc-BSA as an immunogen, and for the obtained rabbit B cells, a test of binding the cell (a single cell) to Siaα2-6Gaβ1-4GlcNAc was carried out by an ELISA method, and 27 positive clones were selected. Antibody genes were obtained from the selected positive clones by single-cell PCR. Each of the obtained antibody gene sequences was transfected into human embryonic kidney cells 293, and the recombinant rabbit antibody secreted into the culture supernatant was subjected to the following ELISA method to check the bindability to the α2,6 sugar chain and the α2,3 sugar chain, thereby obtaining five antibodies (8A2, 8A4, 8B5, 8B9, and 8B19) having high bindability to the α2,6 sugar chain and a low bindability to the α2,3 sugar chain. FIG. 1 shows the results of the ELISA test of these five antibodies.

### [Bindability to α2,6 sugar chain]

50 µl of a Siaα2,6Gal antigen BSA-MBS-peptide conjugate (BSA-MBS-sialic acid α(2,6)β1,4GlcNAc) was added to a 96-well plate at 1 µl/ml (× 2,500) and incubated at 37°C for 1 hour to immobilize the antigen on the well of the plate. After washing 3 times with phosphate-buffered saline (PBS) containing 0.05% Tween 20 (PBST), 100 µl of 1% BSA/PBS was added thereto, and shaking was carried out overnight at 4°C to carry out blocking. After washing 3 times with PBST, 50 µl of the culture supernatant of each clone obtained in Example 1, which had been diluted 2-fold, was added thereto, and shaking was carried out at room temperature for 1 hour. After washing 3 times with PBST, 50 µl of an anti-Rabbit IgG HRP (× 10,000; ab97080, manufactured by abcam, plc) was added thereto, and shaking was carried out at room temperature for 1 hour. After washing with PBST three times, 100 µl/well of a TMB substrate solution (composition: 3.3',5.5'-tetramethylbenzidine, catalog number: N301, manufactured by Thermo Fisher Scientific, Inc.) was added thereto and incubated for 5 minutes. Next, 100 µl/well of 0.1 N HCl was added thereto, and the absorbance at 450 nm was measured.

### [Bindability to α2,3 sugar chain]

The same procedure as above was carried out except that a Siaα2,3Gal antigen BSA-MBS-peptide conjugate was used instead of the Siaα2,6Gal antigen BSA-MBS-peptide conjugate, and the absorbance at 450 nm was measured.

### [Example 2]

Among the monoclonal antibodies obtained in Example 1, the KD values of the 8A2 antibody and the 8B9 antibody were calculated as follows.

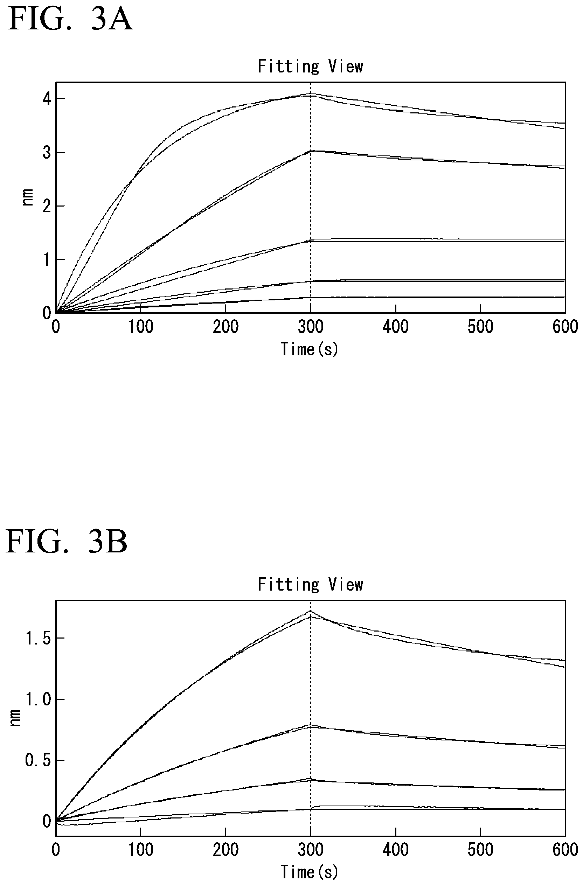

An amino reactive biosensor (AR2G; manufactured by FORTEBIO Inc.) was subjected to hydrophilization with ultrapure water and immersed in a 20 µg/mL BSA-MBS-sialic acid α(2,3)β1,4GlcNAc antigen solution or a BSA-MBS-sialic acid α(2,6)β1,4GlcNAc antigen solution for 10 minutes to immobilize each antigen on the sensor. The sensor was immersed in an EDC-/sNHS solution (composition: mixture of 1-ethyl-3-(3-dimethylaminopropyl)carbodiimide (manufactured by FORTEBIO Inc.) and sulfo-N-hydroxysuccinimide (manufactured by FORTEBIO Inc.) in a ratio of 1:1) to carry out blocking. A BSA-MBS-β1,4GlcNAc antigen immobilized biosensor was prepared by immersing the sensor in a sialidase solution (composition: 3 µL of α(2 → 3,6,8,9) Neuraminidase from Arthrobacter ureafaciens (N3786-1SET, manufactured by Sigma-Aldrich Co. LLC) + 47 µL of 100 mM an acetate buffer (pH 5.5)) at 37°C for 3 hours after blocking to release sialic acid. After washing with PBS, the biosensor was immersed in each antibody solution of 3.75 to 60 nM for 300 seconds under the condition of 30°C, and the binding state of the antibody to the antigen was measured. Then, it was immersed in PBS for 300 seconds, and the dissociation state of the antibody from the antigen was measured. A change in the wavelength shift Δλ caused by the change in the number of antibodies bound to the biosensor or the number of antibodies dissociated from the biosensor was measured in real time to generate a reaction profile on an Octet system (manufactured by FORTEBIO Inc.). KD values were calculated from the sensorgrams of binding and dissociation obtained in antibody solutions at concentrations of 3.75 nM to 60 nM. The calculated KD values are shown in Table 3. In addition, the results of the above sensorgrams of the 8A2 antibody and the 8B9 antibody are each shown in FIG. 2A to FIG. 2C and FIG. 3A to FIG. 3C.

**[Table 3]**

| Antibody name | KD value with respect to α2,6 sugar chain (M) | KD value with respect to α2,3 sugar chain (M) | KD value with respect to galactose-bound sugar chain (M) |
|---|---|---|---|
| 8A2 | 2.9 × 10⁻¹⁰ ± 1.7 × 10⁻²⁴ | 1.3 × 10⁻⁹ ± 1.6 × 10⁻²⁴ | N.D |
| 8B9 | 8.5 × 10⁻⁹ ± 2.7 × 10⁻²⁴ | 9.6 × 10⁻⁷ ± 00 | N.D |

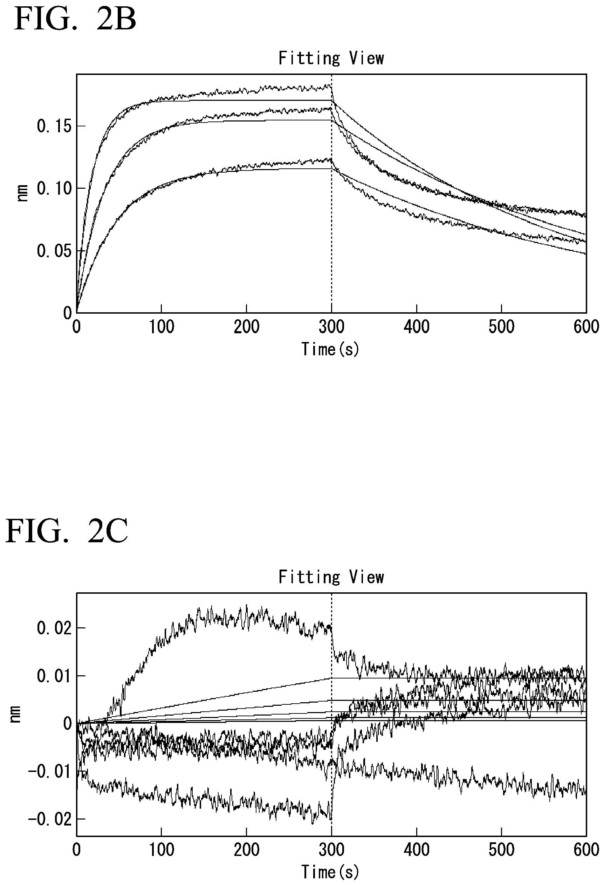

As shown in Table 3, FIG. 2A, and FIG. 2B, the KD value of the 8A2 antibody with respect to the α2,6 sugar chain was 2.9 × 10⁻¹⁰ ± 1.7 × 10⁻²⁴, whereas the dissociation constant KD value with respect to the α2,3 sugar chain was 1.3 × 10⁻⁹ ± 1.6 × 10⁻²⁴. That is, the bindability of the 8A2 antibody to the α2,6 sugar chain was as high as 22.3 times the bindability to the α2,3 sugar chain. Further, as shown in FIG. 2C, no binding to the galactose-bound sugar chain was observed. From these results, it was revealed that the 8A2 antibody is an antibody that has high bindability to the α2,6 sugar chain as compared with the bindability to the α2,3 sugar chain and does not bind to the galactose-bound sugar chain.

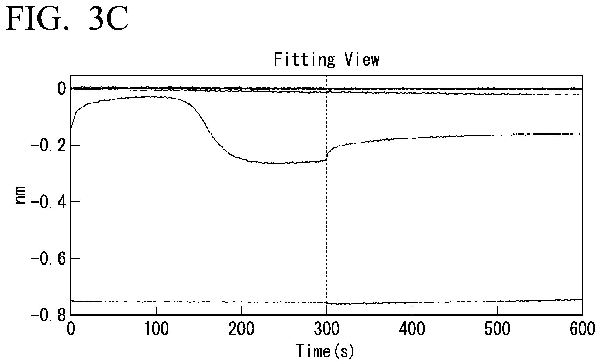

In addition, as shown in Table 3, FIG. 3A, and FIG. 3B, the KD value of the 8B9 antibody with respect to the α2,6 sugar chain was 8.5 × 10⁻⁹ ± 2.7 × 10⁻²⁴, whereas the KD value with respect to the α2,3 sugar chain was 9.6 × 10⁻⁷ ± 00. That is, the bindability of the 8B9 antibody to the α2,6 sugar chain was as high as 88.5 times the bindability to the α2,3 sugar chain. Further, as shown in FIG. 3C, no binding to the galactose-bound sugar chain was observed. From these results, it was revealed that the 8B9 antibody is an antibody that has high bindability to the α2,6 sugar chain as compared with the bindability to the α2,3 sugar chain and does not bind to the galactose-bound sugar chain.

### Industrial Applicability

According to the present invention, a monoclonal antibody or an antibody fragment thereof having high bindability to an α2,6 sugar chain, and a method for measuring an α2,6 sugar chain are provided.

## Claims

1. A monoclonal antibody or an antibody fragment thereof which specifically binds to a sugar chain in which a terminal sialic acid residue is bound to galactose with α2,6 linkage, and which does not bind to a sugar chain to which galactose having no sialic acid bound to a terminal is bound.

2. The monoclonal antibody or the antibody fragment thereof according to Claim 1,
wherein a dissociation constant with respect to the sugar chain in which the terminal sialic acid residue is bound to galactose with α2,6 linkage is 1.0 × 10⁻⁸ or less.

3. The monoclonal antibody or the antibody fragment thereof according to Claim 1 or 2,
wherein the bindability to the sugar chain in which the terminal sialic acid residue is bound to galactose with α2,6 linkage is 20 times or more with respect to the bindability to a sugar chain in which a terminal sialic acid residue is bound to galactose with α2,3 linkage.

4. The monoclonal antibody or the antibody fragment thereof according to any one of Claims 1 to 3,
wherein an amino acid sequence of a complementarity determining region (hereinafter, also referred to as CDR) 1 of a heavy chain variable region (hereinafter, also referred to as VH) of the antibody or the antibody fragment thereof comprises an amino acid sequence set forth in SEQ ID NO: 1, an amino acid sequence of CDR2 of the VH comprises an amino acid sequence set forth in SEQ ID NO: 2, an amino acid sequence of CDR3 of VH comprises an amino acid sequence set forth in SEQ ID NO: 3, an amino acid sequence of CDR1 of a light chain variable region (hereinafter, also referred to as VL) comprises an amino acid sequence set forth in SEQ ID NO: 4, an amino acid sequence of CDR2 of VL comprises an amino acid sequence set forth in SEQ ID NO: 5, and an amino acid sequence of CDR3 of VL comprises an amino acid sequence set forth in SEQ ID NO: 6.

5. The monoclonal antibody or the antibody fragment thereof according to Claim 4,
wherein an amino acid sequence of the VH of the antibody or the antibody fragment thereof comprises an amino acid sequence set forth in SEQ ID NO: 13, and an amino acid sequence of VL comprises an amino acid sequence set forth in SEQ ID NO: 14.

6. The monoclonal antibody or the antibody fragment thereof according to any one of Claims 1 to 3,
wherein an amino acid sequence of CDR1 of the VH of the antibody or the antibody fragment thereof comprises an amino acid sequence set forth in SEQ ID NO: 7, an amino acid sequence of CDR2 of the VH comprises an amino acid sequence set forth in SEQ ID NO: 8, the amino acid sequence of CDR3 of VH comprises an amino acid sequence set forth in SEQ ID NO: 9, an amino acid sequence of CDR1 of VL comprises an amino acid sequence set forth in SEQ ID NO: 10, an amino acid sequence of CDR2 of VL comprises an amino acid sequence set forth in SEQ ID NO: 11, and the amino acid sequence of CDR3 of VL comprises an amino acid sequence set forth in SEQ ID NO: 12.

7. The monoclonal antibody or the antibody fragment thereof according to Claim 6,
wherein an amino acid sequence of the VH of the antibody or the antibody fragment thereof comprises an amino acid sequence set forth in SEQ ID NO: 15, and an amino acid sequence of VL comprises an amino acid sequence set forth in SEQ ID NO: 16.

8. A method for measuring a sugar chain in which a terminal sialic acid residue is bound to galactose with α2,6 linkage, the method comprising:
using the monoclonal antibody or the antibody fragment thereof according to any one of Claims 1 to 7.
